# EUROPEAN PATENT SPECIFICATION

(11) **EP 2 755 617 B1**
(45) Date of publication and mention of the grant of the patent: **04.07.2018**
(21) Application number: 12832234.4
(22) Date of filing: 11.09.2012
(51) Int. Cl.: A61N 1/05

(54) **METHOD FOR FABRICATION BIOCOMPATIBLE ELECTRODE COMPONENT**
HERSTELLUNGSVERFAHREN FÜR BIOKOMPATIBLE ELEKTRODENKOMPONENTE
PROCÉDÉ DE FABRICATION D'UN COMPOSANT D'ÉLECTRODE BIOCOMPATIBLE

(30) Priority: 13.09.2011 US 201161533910 P; 13.09.2011 AU 2011903731
(43) Date of publication of application: 23.07.2014
(73) Proprietor: HEAR IP PTY LTD, Melbourne, Victoria 3010 (AU)
(72) Inventor: MERGEN, Silvana, Brighton, Victoria 3186 (AU); NEWBOLD, Carrie, Eaglemont, Victoria 3084 (AU)
(74) Representative: Walker, Ross Thomson
(86) International application number: PCT/AU2012/001083
(87) International publication number: WO 2013/036988

(56) References cited:
- WO-A1-99/45375
- WO-A2-00/25854
- WO-A2-2007/146082
- WO-A2-2009/006636
- JP-A- 2011 097 024
- US-A1- 2010 198 301
- US-A1- 2010 305 673
- US-A1- 2011 130 815
- M SCHUETTLER ET AL.: 'Fabrication of implantable microelectrode arrays by laser cutting of silicone rubber and platinum foil' JOURNAL OF NEURAL ENGINEERING vol. 2, 2005, pages S121 - S128, XP020093418
- T. A. FOFONOFF ET AL.: 'Microelectrode Array Fabrication by Electrical Discharge Machining and Chemical Etching' IEEE TRANSACTIONS ON BIOMEDICAL ENGINEERING vol. 51, no. 6, June 2004, pages 890 - 895, XP011113162
- JIAN WU: 'Micromachined Auditory Prosthetic Devices: Design, Fabrication and Integration' DOCTORAL DISSERTATION, UNIVERSITY OF CALIFORNIA 2006, XP055151904

## Description

### FIELD OF THE INVENTION

The present invention relates generally to electrodes, and methods for fabricating same. In particular, the present invention relates to electrodes that are useful for providing electrical stimulation or sensing electrical activity in biological tissues.

### BACKGROUND TO THE INVENTION

Electrical signals are important mediators in many biological systems, and especially so in nervous systems of animals. For example, skeletal muscle contraction is instigated by the depolarisation of cells originating in the central nervous system, which propagates through to peripheral nerves membrane in electrical communication with the muscle.

Important sensory functions are dependent upon the relay of electrical currents from peripheral nerves to the central nervous system. For example, the stimulation of photoreceptor cells in the eye activates neurons of the optic nerve which in turn activate cells of the visual cortex in the brain. Similarly, sound waves mechanically stimulate the hair cells of the cochlea which convert the sound waves into electrical signals which are carried to the auditory cortex of the brain to provide hearing.

There are many acquired conditions and genetic defects which result in the impaired relay of electrical signals in biological systems. Such defects may result in paralysis, blindness or deafness. The biomedical engineering arts have attempted to remedy such defects by artificially stimulating various biological tissues using implantable electrodes of various types. One eminently successful application has been in the treatment of deafness using cochlear electrode implants.

In addition to the stimulation of tissues with electrical current, it is also necessary to sense electrical activity within the body. In certain investigative fields (such as the neurosciences) it is desirable to monitor electrical activity about a biological tissue with a view to understanding underlying electrophysiological mechanisms. This end may be achieved by the implantation of electrodes into a tissue to measure potential differences across a cell, tissue, organ or even an entire organism.

As will be appreciated, electrodes for use in biological applications must be fabricated accurately, and often in very small dimensions in order to specifically contact a target tissue. As an example, a cochlear electrode array must be capable of insertion into the confined spaces of the labyrinth, and to also present electrodes in register with the tonotopical organization of the organ.

The prior art discloses many methods for the fabrication of electrodes for use in a biological setting. A widely used method relies on complex photolithographic methods to lay down channelsthat ultimately receive a conductive electrode material.

These methods rely on the use of toxic substances such as a photoresist compounds, solvents and developers. Given that the electrodes subject of this application may be implanted into a human, it is dearly necessary to remove all such compounds before use, this requiring additional steps in the fabrication process. Furthermore, some of the compounds used in photolithography may react with certain components of the electrode leading to adverse chemical, structural or functional changes. Such compounds also provide a clear occupational health and safety risk to persons involved in the fabrication of electrodes.

Many non-photolithographic methods for fabricating cochlear electrodes also require the use of chemicals at one or more steps in the fabrication process. For example, organic solvents such as chloroform are used to deposit and/or remove materials from the electrode during fabrication. Such solvents are problematic since residues can remain on the electrode.

The presence of residues is a significant problem in medical device fabrication. In particular, it is necessary to select a residue detection method, electing a sampling method, set residue acceptance criteria, provide residue removal validation methods and implement recovery studies, prepare written procedures for removal and train facility staff accordingly. The further regulatory work required is substantial and can add cost to the development of a medical device. In some cases, it may not even be possible to remove the residue to an acceptable level. The occupational health and safety issues described supra are also relevant.

Photolithographic methods for fabrication may also result in electrodes having practical shortcomings. For example, these methods are generally capable of providing electrode films of only limited thickness leading to insufficient conductance in some applications. Another deficiency is that the electrodes may exhibit a limited surface area, thereby requiring larger than desirable electrode pads to achieve a given level of stimulation to a tissue. The use of larger electrodes is contraindicated in cochlear implants because of the need to stimulate very small and specific areas within the cochlea.

It is an aspect of the present invention to overcome or alleviate a problem of the prior art by providing alternative or improved methods for fabricating electrodes useful for biological applications. It is a further aspect to provide alternative or improved electrodes *per se.*

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

WO 2007/146082 A2 relates to a process for manufacturing moulded polymer comprising silicone and at least one metal trace. WO 2007/146082 A2 shares the features of the preamble of claim 1.

### SUMMARY OF THE INVENNTION

The present invention provides a method for fabricating an electrode device component, the method comprising the steps of:
(i) providing a biocompatible carrier material having a protective layer and
(ii) performing an ablative method on the biocompatible carrier material to form a recess, the recess capable of receiving a biocompatible electrode material having a thickness of at least about 1 micron,
(iii) removing the protective layer, and
(iv) depositing a conductive material having a thickness of at least about 1 micron into the recess of the carrier material,
wherein the ablative method does not require exposure of the carrier material to a chemical compound; and
characterised in that the protective layer is provided by applying a pre-formed film to the biocompatible carrier material. In one embodiment, the ablative method does not require exposure of the carrier material to certain chemical compounds, and particularly any of the toxic chemical compounds used in prior art photolithograhy or microstructuring methods such as photoresists, developers,
removers, strippers, thinners, EBR, adhesion promotion agents and ancillary chemicals, etchants, and solvents.

In another embodiment, the ablative method does not require exposure of the carrier material to a solution used in the application and/or removal of a protective layer deposited on the carrier material. The solvent of the solution may be an organic solvent including chloroform.
In one embodiment the ablative method relies on heating the carrier material, and may be a a laser ablative method. In particular, the laser ablative method may comprise use of an excimer laser.

In one embodiment, the recess formed by the ablative method is of sufficient depth to allow the deposition of an electrode film of between about 1 and 25 micron thickness. In one embodiment the recess formed by the ablative method is of sufficient depth as to allow the deposition of an electrode film of at least about 1 micron thickness.

In one embodiment of the method the biocompatible carrier material has, or is provided with, a protective layer. Accordingly, the method may comprise the step of applying the protective layer to the carrier material after the step of providing the carrier material and before the step of performing the ablative method. The step of applying may be accomplished without the use of a solvent, and particularly an organic solvent such as chloroform.

The protective layer may be a film, and in one embodiment has a thickness of less than about 10 micron, and particularly from about 5 to 7 micron. In one embodiment, the film is pre-formed, and applied to the biocompatible carrier. The film may have an adhesive to facilitate attachment of the film to the carrier. The film may be a polymeric film, such as a polyimide.

In one embodiment the method comprises the step of removing the protective layer after performance of the ablative method. The protective layer may be removed without the use of a solvent, and particularly an organic solvent such as chloroform.

In another embodiment the method comprises the step of depositing a conductive material into the recess of the carrier material. The conductive material may be deposited using a vapour deposition method such as a sputtering method.

A further embodiment of the method comprises the step of masking the carrier material prior to deposition of the conductive material. The method may comprise the step of applying a shadow mask to the carrier material prior to deposition of the electrode material. The shadow mask may have a thickness of between about 25 and 75 microns, and in certain embodiments is fabricated from a laser machinable material.

Described herein is an electrode component comprising an electrode material portion and a carrier material portion wherein the carrier material portion is of unitary construction.

Described herein is an electrode component comprising an electrode material portion and a carrier material portion wherein the surface of the electrode material portion has an increased level of roughness relative to the level of roughness of a similar electrode produced by a photoresist method.

Also described herein is a multi electrode array for use in a cochlear implant, the array comprising between about 22 to 32 electrodes.

Described herein is an electrode component comprising an electrode material portion and a carrier material portion wherein the electrode material portion has a geometric surface area of less than about 0.03 mm², 0.0299 mm², 0.02298 mm², 0.02297 mm², 0.02296 mm², or 0.02295 mm².

Also described is a multi electrode array for use in a cochlear implant, the array comprising a plurality of electrode pads, the distance between two pads being less than about 0.7mm.

Described herein is an electrode component comprising an electrode material portion and a carrier material portion wherein the electrode material portion has a thickness of between about 1 to 20 micron.

Described herein is an electrode component produced by a method as described herein. In some embodiments of the invention, the electrode component has one or more of the following properties: the carrier material portion is of unitary construction, the surface of the electrode material portion has an increased level of roughness relative to the level of roughness of a similar electrode produced by a photoresist method, the electrode material portion has a geometric surface area of less than about 0.03 mm², the electrode material portion has a thickness of between about 1 to 20 micron.

Also described is a multi-electrode array produced by a method as described herein. In some embodiments of the invention, the multi-electrode array has one or both of the following properties: the array comprising between about 22 to 32 electrodes, the array comprising a plurality of electrode pads, the distance between two pads being less than about 0.7mm. Described herein is an implantable electrode device comprising an electrode component as described herein; or a multi-electrode array as described herein. In one arrangement the implantable elect rode device is a cochlear implant.

Further, described herein is a method of treating, preventing or ameliorating a condition associated with aberrant electrical stimulation of a tissue, the method comprising the step of implanting an electrode component as described herein; a multi-electrode array as described herein; or an implantable electrode device as described herein into an animal in need thereof. In one arrangement of the method the condition associated with aberrant electrical stimulation of a tissue is deafness.

Examples, aspects and embodiments disclosed herein, but not falling under the scope of claim 1, do not form part of the invention.

### BRIEF DESCRIPTION OF THE FIGURES

Fig. 1 is a diagram of a preferred method of the invention. The white area represents the silicone elastomer, the black area represents the glass substrate, the forward-leaning shading represents the Kapton™ tape, the backward-leaning shading represents the brass mask.
Fig. 2 is a graphical comparison of the potential transients for three types of electrodes. The black line represents an electrode that was not laser machined, the pink line represents laser machined electrode (designated "design A"), the redline represents laser machined electrode (designated "design B"), while the green line represents a guinea pig electrode.
Fig 3. is a graphical representation of potential transients of laser machined square pads (designated "electrode 8") before and after 8 weeks of continuous stimulation. Diamond-shaped data points represent cathodic Va, Square-shaped data points represent cathodic Vt, triangular data points represent anodic Va. Cross-shaped data points represent anodic Vt.

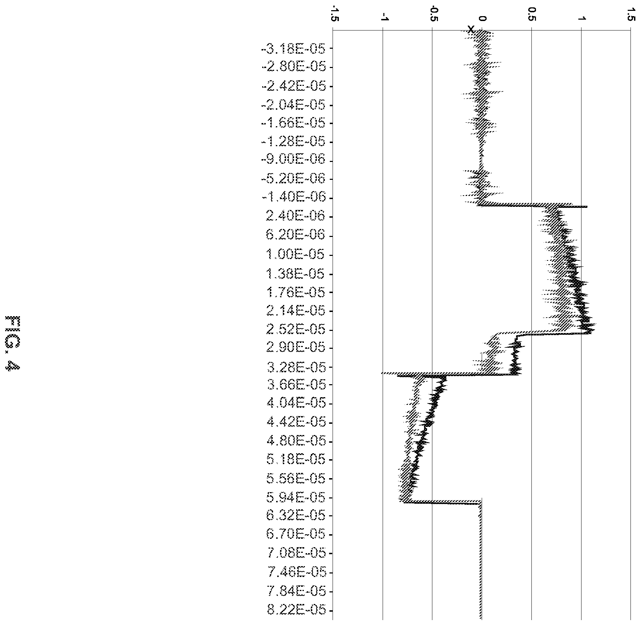

Fig. 4 is a graphical comparison of the biphasic potential transient measurement for a thin film electrode made using photolithography on glass substrate (blue graph) and thick film electrode employing laser machining of silicone (pink graph).
Fig. 5 is an electron micrograph showing 5 electrode pads and tracks on a silicone substrate.
Fig 6. is a higher magnification of the upper electrode pad shown in Fig 5 demonstrating surface roughness of the thick film.
Fig. 7 is an electron micrograph showing the edge of an electrode pad with some silicone exposed showing the topography of the laser machined silicone and the platinum film when deposited on a silicone substrate.
Fig. 8 is an electron micrograph showing the edge of an electrode pad covered with platinum thick film. Some of the laser machined silicone substrate is exposed.
Fig. 9 isa higher magnification of the bumps in the platinum thick film shown in Fig. 8.
Fig. 10 is an electron micrograph showing an electrode pad and track with some areas of the laser machined substrate not covered by the thick film. The bright areas represent platinum with the darker rim around the pad showing the exposed laser machined silicone substrate.
Fig. 11 is an electron micrograph of a track showing a width of around 59 micron.
Fig. 12 isan electron micrograph showing platinum film deposited on a glass substrate.
Fig. 13 is an electron micrograph showing platinum film deposited on a laser machined silicone substrate.
Fig. 14 is an electron micrograph showing platinum film deposited on a silicone substrate that was not laser machined.
Fig. 15 is an electron micrograph showing a cross section of platinum film deposited on a silicone substrate.

### DETAILED DESCRPTION OF THE INVENTION

After considering this description it will be apparent to one skilled in the art how the invention is implemented in various alternative embodiments and alternative applications. However, although various embodiments of the present invention will be described herein, it is understood that these embodiments are presented by way of example only, and not limitation. As such, this detailed description of various alternative embodiments should not be construed to limit the scope or breadth of the present invention. Furthermore, statements of advantages or other aspects apply to specific exemplary embodiments, and not necessarily to all embodiments covered by the claims. Various embodiments and advantages of the present invention will be described mainly by reference to electrodes for use in cochlear electrode arrays, however it will be understood that such disclosure is nonlimiting and that application to other types of electrode devices contemplated.

Unless the contrary intention is expressed, the features presented as preferred or alternative forms of the invention can be present in any of the inventions disclosed as alone or in any combination with each other.

Throughout the description and the claims of this specification the word "comprise" and variations of the word, such as "comprising" and "comprises" is not intended to exclude other additives, components, integers or steps.

The present invention is at least partially predicated on the Applicant's finding that electrode device components can be fabricated using ablative techniques, and that such techniques provide alternatives to current methods and electrodes, and in some instances provide an advantage over those known at the filing date of this application.

The present invention provides a method for fabricating an electrode device component, the method comprising the steps of
(i) providing a biocompatible carrier material having a protective layer and
(ii) performing an ablative method on the biocompatible carrier material to form a recess, the recess capable of receiving a biocompatible electrode material having a thickness of at least about 1 micron,
(iii) removing the protective layer, and
(iv) depositing a conductive material having a thickness of at least about 1 micron into the recess of the carrier material,
wherein the ablative method does not require exposure of the carrier material to a chemical compound; and
characterised in that the protective layer is provided by applying a pre-formed film to the biocompatible carrier material. Applicant's direct yet effective approach of forming a recess directly into the carrier material is a significant departure from prior art methods. The use of an ablative method as described herein may provide one or more advantages in the fabrication process. For example, it will be clear that the present methods are less complex and more economical, requiring significantly less labour and consumable chemicals.

The lowered complexity of the present methods also affords amenability to automation or partial automation of the fabrication process. As will be appreciated by those skilled in the art, the fabrication of many types of implantable electrode is achieved to a large extent by hand in a clean room environment. An advantage of the method is that the ablative method does not require exposure of the carrier material to certain chemical compounds. As mentioned,
prior art methods for microstructuring and lithography often require the use of toxic chemicals. Such chemicals include photoresists, developers, removers, strippers, thinners, EBR adhesion promotion agents and ancillary chemicals, etchants, and solvents (such as acetone and toluene). Exemplary chemicals for which the present methods are not reliant are referred to by reference to the trade names used by MicroChemicals GmbH, Schillerstrasse 18, D-89077 Ulm, Germany, and include :AZ®111 XFS, AZ®1505, AZ®1512 HS, AZ®1514 H, AZ®1518, AZ®4533, AZ®4562, AZ®4999, AZ®5214 E, AZ®6612, AZ® 6632, AZ®9260, AZ®ECI 3027, PL®177, AZ®MIR701, AZ®15 nXT, AZ®125 nXT, AZ®40 XT, AR®nLOF 2070; AZ®nLOF 2070 AZ®520 D, MicroChemicals TI 35 E, MicroChemicals TI 35 ES, MicroChemicals TI xLift, MicroChemicals TI Rating, MicroChemicals TI Prime, MicroChemicals TI Spray.

Some prior art methods require the application and/or removal of a protective layer to the biocompatible carrier at some stages of the fabrication process. Such protective layers are formed *in situ* on the surface of the carrier by the application of a solution, with evaporation of the solvent leaving to deposition of the solute on the surface of the carrier as a protective layer. Reference is made to the methods of Dupas-Bruzek et al; J. Appl. Phys. 106, 074913 (2009), whereby a solution of poly(methylmethacrylate) in chloroform is sprayed onto a polydimethylsiloxane substrate. Later in the fabrication process, the poly(methylmethacrylate) (PMMA) layer is dissolved by washing the substrate in chloroform. The present methods obviate the need for exposure of the substrate to solvents such as chloroform.

Chloroform is hazardous where contacted on the skin, and eye. It is a proven carcinogen: classified + (Proven.) by NIOSH; classified A3 (Proven for animal.) by AOGIH, 2B (Possible for human.) by IARC; classified 2 (Some evidence.) by NTP. Chloroform has some mutagenic effects as shown in mammalian somatic cells, bacteria and/or yeast. The substance may be toxic to kidneys, liver, heart. Repeated or prolonged exposure to the substance can produce target organ damage.

Similarly PMMA is known to cause skin irritation and eye irritation. PMMA is listed as an inhalation hazard, and also has an ability to target the liver, kidneys, bladder and brain. The compound is listed as equivocally tumorigenic by RTECS.

Thus, in obviating the need to expose the biocompatible carrier to compounds that may have an adverse effect on a recipient of the electrode, the present invention provides significant advantage. Further advantages of the present invention will become evident upon consideration of the further disclosure *infra.*

In one embodiment, the method is devoid of the step of performing a surface modification on the ablated surface. In some methods of the prior art, it is necessary to specially modify the surface of the recess in order to facilitate attachment of conductive materials such as metals. The ablative methods described herein (optionally in combination with the deposition methods described herein) allow for a simpler electrode fabrication process that does not require any discrete surface modification step.

The present methods are useful in the fabrication of electrode device components. As will be readily understood by the skilled person a complete and functional electrode device is typically comprised of a number of components including the biocompatible electrode material which forms the electrical connection with the biological tissue, as well as ancillary components such as carrier structures to support the conductive electrode pad, wires, housings and the like.

The method described *supra* is directed to the fabrication of a substantially non-conductive carrier structure to support the conductive biocompatible electrode material. In this capacity, the carrier material may act to properly orient the electrodes relative to each other, or relative to some other part of the electrode device such as a housing. The correct positioning and spacing of electrode pads in the multi-electrode arrays of cochlear implants is important in ensuring that the correct regions of the cochlea are stimulated, this in turn leading to more accurate sound reproduction.

Alternatively or in addition to a role as a supporting structure, the carrier may also act as a mould in forming a biocompatible electrode material that is applied to the carrier structure in a subsequent step. The electrode material may be deposited into the recess formed in the carrier material, where it bonds to the carrier material. Typically, the recess has a broad and shallow configuration such that deposition of an electrode material therein provides a planar structure such as a pad. It will be understood nevertheless that the recess may be shaped otherwise such that other conductive structures such as wires, connection termini and the like are amenable to fabrication according to the present methods.

As will become apparent after reading the entire disclosure of this specification, the present methods may comprise further steps required to provide a functional electrode device. In particular, some embodiments of the method comprise step(s) required for the application of conductive materials to the carrier structure, as further described *infra.* However, it will be understood that in one embodiment the method is directed only to the fabrication of a carrier structure having a recess.

Suitable carrier materials useful in the present methods include biocompatible polymers such as silicones, polyimides, polyvinylidine fluorides, polyphenylsulfones, poly(p-xylylene) polymers and the like. Of the silicones, an exemplary class is the Silastic type, and in particular the MDX 4-4210, MED 4830, MED4 860 types. Suitable poly(p-xylylene) polymers are those sold under the trade name of Paralyne (Para Tech Coating Inc, Aliso Viejo, CA). The skilled person is familiar with a number of other biocompatible carrier materials suitable for use in the present invention, with all being included in the scope of this application.

As used herein, the term "biocompatible" is intended to mean that the material is able to contact a biological tissue without negatively affecting the function of that tissue (or indeed the entire organism) in any substantive manner. The specific requirements for biocompatibility will of course vary according to the application concerned. For example, electrode components that are designed for only short term use (such as in the context of interventional cardiology), may not need to account for the possibility of host rejection. However, such responses may be a significant issue for components that are intended for long term implantation such as cochlear implants, or electrodes used in deep brain stimulation.

Against the background of known photoresist methods used in the fabrication of electrode components, Applicant proposes a divergent method. It has been found that biocompatible materials (such as silicones) that are used for carriers of conductive electrode materials can be formed by an ablative method whereby a recess is directly formed into the carrier material. The conductive electrode material is then subsequently deposited into the so-formed recess.

As used herein, the term "ablative method" is intended to mean any method that is capable of removing a portion of the biocompatible carrier material in a controlled manner leading to a recess having a predetermined dimension. The use of an ablative method is in direct contrast to the well accepted and used photolithographic methods that rely on a photoresist compound to form a walled structure that extends beyond the surface of the carrier material. After the deposition of a conductive electrode material to the patterned carrier material, the walled structure form by the photoresist is removed to expose the deposited conductive material. As will be appreciated, the deposited conductive material sits proud of the carrier surface, and so further carrier material must be moulded about the conductive material to protect the edges of the electrode. The excess further carrier material must be removed in a separate process step to leave the electrode surface properly exposed. By contrast, the present methods are dearly simpler by requiring a single ablative step in order to generate the recess into which the conductive material is deposited.

Ablative methods useful in the context of the present invention generally rely on an erosive process such as vapourising, conversion to a plasma, melting, burning, freezing, chipping, etching, dissolving, abrading, or subliming the carrier material to form a recess.

In a preferred embodiment of the invention the ablative method does not require exposure of any chemical (and in particular any toxic chemical) to the carrier material. An example of thistype of ablation is vaporisation whereby extreme heating of the carrier material leadsto conversion of the material to a vapour or gas.

In one embodiment, the ablative method is a laser ablative method. It has been found that laser ablation is particularly suited to the present methods given the high level of control provided by the laser beam. Considerable accuracy can be provided as to the position and dimensions of the recess when ablated by laser-based techniques. The depth to which the laser energy is absorbed, and thus the amount of material removed by a single laser pulse, can be closely controlled.

For some laser ablative methods performed at low laser flux, the carrier material is heated by the absorbed laser energy and evaporates or sublimates. At high laser flux, the material may be converted to a plasma.

In a preferred form of the method, the laser used in the ablative method emits light energy at a frequency that does not substantially heat the biocompatible carrier material. Alternatively or additionally, the laser is operated under conditions such that the biocompatible material is not substantially heated.

In one form of the method, the laser emits light energy in the ultraviolet range. A particularly preferred laser is the excimer laser (also known as an exciplex laser). The light from an excimer laser is well absorbed by many biocompatible carrier materials that are useful in the context of the present invention. Rather than burning or cutting the biocompatible carrier material, the excimer laser emits sufficient light energy to disrupt molecular bonds of the carrier material surface. The carrier material is effectively disintegrated in a controlled manner, with the disintegrated material being propelled into the surrounding environment. Excimer lasers are particularly useful in the present methods since they are capable of removing fine layers of carrier surface material with minimal heating or other alteration to non-target material which is left substantially intact.

The wavelength of the excimer laser relies on the partner excimer molecule used. In certain forms of the method the excimer is selected from the group consisting of Ar₂ (126 nm), Kr₂ (146 nm), Xe₂ (172, 175 nm), ArF (193 nm), KrF (248 nm), XeBr (282 nm), XeCl (308 nm), XeF (351 nm), and KrCl (222 nm).

In a preferred form of the method, the laser is an ArF excimer laser. This laser operates at a wavelength of 193 nm, and is particularly suited to the ablation of silicone carrier materials.

While some routine experimentation may be required to optimise the ablative method, the skilled person is able to select an appropriate laser (and laser wavelength) based on the optical properties of the carrier material.

Laser ablation may include the removal of carrier material with a pulsed laser, or a continuous wave laser beam. Where a pulsed laser is used the pulse time may be routinely varied over the operable range of the laser to achieve a recess having the required dimensions.

In one embodiment of the method, the recess formed by the ablative method is of sufficient depth as to allow the deposition of an electrode film of at least about 1 micron thickness, and preferably at least about 2, 3, 4, 5, 6, 7, 8, 9, 10, 11, 12, 13, 14, 15, 16, 17, 18, 19, 20, 21, 22, 23, 24, 25,26, 27, 28, 29, 30, 31, 32, 33, 34, 35, 36, 37, 38, 39, 40, 41, 42, 43, 44, 45, 46, 47, 48, 49, or 50 micron thickness. In one embodiment, the recess has a depth of about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 micron. For many applications a recess of about 10 micron in depth is used to provide an electrode film thickness of 10 micron.

The shape of the recess may be varied according to the end function of the carrier material or any electrode device incorporating the carrier material. Typically, the walls and floor of the recess will be substantially planar in geometry however it will be understood that some applications may require curved geometries, or possibly even irregular morphologies. As an example, a floor having a curved geometry may be advantageous in the fabrication of electrode arrays incorporated into cochlear implants given the geometry of the scala tympani of the cochlea.

By contrast to the present methods, the prior art photolithography methods are practically useful for allowing thin film deposition of only up to 1 micron thickness. To achieve acceptable results in photolithography, the thickness of the photoresist layer must be significantly greater than the thickness of the deposited electrode film. Thus, to obtain even a modest increase in the thickness of the electrode film it is necessary to dramatically increase the thickness of the resist layer. The laying down of very thick photoresist layers is exceedingly difficult technically and often unsuccessful. By contrast, the ablative methods allow for a relatively deep recess to be formed in the carrier material. This allows for the deposition of a much thicker electrode film, an advantage of the invention which is further discussed *infra.*

In one embodiment of the invention, the biocompatible carrier material has, or is provided with, a protective layer. As used herein the term "protective layer" is intended to include a layer of any material that is capable of (i) adhering to the surface of the carrier material, and remain adherent during the ablation step; and (ii) prevent any contaminants (such as debris, spatter or other by-product) produced by the ablative method from falling back onto the surface of the carrier material. It will be appreciated that such contaminants may be toxic, or may adversely affect a physical or chemical property of the carrier material.

As will be apparent, the protective layer will be of a thickness and composition allowing ablation of the underlying material. Thus, where the ablative method is laser-based for example, the protective layer must be amenable to laser ablation, allowing ablation of the underlying carrier material.

The protective layer may be incorporated into the carrier material during manufacture of material, or indeed may be inherent in the carrier material. In one embodiment, the protective layer is simply an outer region of the carrier, and is chemically and physically indistinct and continuous with the remainder of the carrier. In this case the protective layer is removed from the underlying carrier material by any appropriate method, including by use of a microtome.

The protective layer may be of the same material as the carrier material, and incorporated during manufacture of the carrier. For example, when the carrier material is fabricated, a dividing element (such as a thin polymer film) may be embedded within the material to define an outer protective layer. After laser ablation the dividing element is lifted taking away the protective layer (along with any contaminants), leaving an uncontaminated carrier surface.

In place of a physical dividing element as described *supra,* the carrier material may be fabricated to contain a plane of structural weakness. In that case, after laser ablation the plane of structural weakness is exploited to separate the upper protective layer from the underlying bulk carrier material.

Alternatively, the carrier material may be fabricated in two distinct layers, with a thin protective layer fabricated over a preformed underlying bulk of carrier material. In this embodiment the protective layer may be composed of the same material as the underlying bulk carrier material, or may be composed of a different material altogether.

In one embodiment, the protective layer is applied to the carrier material in a manner completely distinct from the manufacture of the carrier material. One manner in which that end may be achieved is by application of a thin film to the carrier material subsequent to manufacture. The thin film may be adhered to the carrier material by any means including an adhesive or a physical force (such as *Van der Waals* force, or static attraction). Any thin film and adhesive used must of course be amenable to removal by the ablation method concerned. Furthermore, any adhesive must provide a sufficient level of adherence to prevent lifting from the carrier material, but is not so adherent that the film is difficult to remove, or causes gross damage to the carrier material upon removal. A further desirable property of any adhesive is that it leaves little or no residue on the carrier material after removal. In embodiments of the method whereby an adhesive residue remains, cleaning in an ultrasonic bath and isopropanol for 30 minutes may be sufficient for removal. Mechanical removal with a cotton swab may also be useful. In one embodiment, the method of deaning does not require the use of a chemical, or a toxic chemical (for example an organic solvent such as chloroform).

In one embodiment of the method the adhesive is a silicone adhesive which is spin coated onto the surface of the carrier material, with the thin film protective layer being applied onto the adhesive.

In one embodiment of the method, the thin film used as a protective layer is a polymeric film or tape having a thickness of less than about 10, 9, 8, 7, 6, 5, 4, 3, 2, or 1 micron. A particularly suitable film or tape is a polyimide having a thickness of from about 5 to 7 micron, and preferably about 6 micron. In a highly preferred form of the method, the protective layer is a commercially available polyimide tape that includes an adhesive backing. Such tapes are sold under the trade name of Kapton™ which is a polyimide film developed by DuPont. Kapton™ tape is supplied by RScomponents Pty Ltd, Smithfield, New South Wales, Australia ; Sock No. 383-3960. This specific Kapton™ tape has a silicone adhesive of thickness about 30 to 40 micron, with the polyimide layer also being about 30 to 40 micron thick.

The inventive methods provided *supra* lead to the formation an electrode component comprising a biocompatible carrier structure having a recess of defined dimensions. As will be noted, such a component is devoid of a conductive material. Accordingly, in certain embodiments the present method comprises the step of depositing a conductive material into the recess of the carrier material.

The skilled person is capable of selecting an appropriate electrode material for the intended application of the electrode component. The skilled person is very familiar with such materials, and they include platinum, iridium, titanium, gold, silver, iridium oxide, rhodium, rhodium oxide, tantalum, titanium nitride, niobium and any alloys thereof. Composite electrodes such asthose combining platinum and iridium are also contemplated.

The skilled person is familiar with a number of methods for depositing metal electrode materials onto biocompatible carrier materials. For example, physical vapour deposition methods (such as sputtering techniques) which can be used to deposit metals onto substrates are well known to the skilled person, as illustrated by the following publications, D.M.Mattox, Handbook of physical vapour deposition (PVD) processing, (1998) Noyes Publication, New Jersey T. Stieglitz, Electrode Materials for recording and stimulation, Ed. Kenneth W Horch and Gurpreet S Dhillon, Neuroprosthetics Theory and Practice, Series of Bioengineering & Biomedical Engineering Vol.2, World Scientific, 2004, New Jersey.

Applicant proposes that advantages are provided by using sputtering methods in the fabrication of electrodes, such as cochlear electrodes. Such methods result in the deposition of dense films with substantially reproducible structure, and formation of minimal or no pores. With respect to Platinum at least, the deposited films are of very high purity. Furthermore, the hardness of the films in the thickness direction is the same or greater as that shown by bulk platinum (as characterised through nanoindentation measurements).

Sputtered coatings are also resistant against corrosion, this being important in biological environments which are typically moist and salty.

Sputtered coatings deposited onto an ablated surface advantageously provide a surface roughness, which can be controlled in the present methods. Roughness is an important parameter as it directly relates to the surface area available to exchange electrons at the electrode/ tissue interface. Controlling the effective electrode area is a key requirement for the safe use of electrodes in human recipients.

Given the aforementioned publications, other similar publications in the field, and the knowledge of the skilled person, it will be possible by no more than routine experimentation to arrive at a suitable sputtering method. In one embodiment of the method, magnetron DC sputtering is used. It may be necessary to alter standard parameters where silicone is used to prevent degradation or crazing of the deposited metal film. For example, it may be necessary to coat at a lower temperature, lower bias and higher pressure than usual to provide optimal deposition on the film.

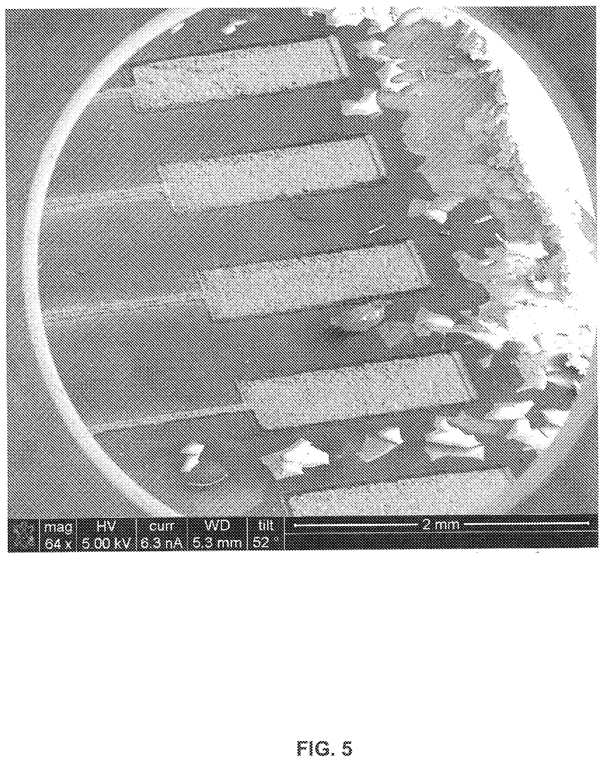

A number of companies provide physical vapour deposition services on a fee-for-service basis, and are capable of performing any routine optimization studies for a given application. One example is Teer Coatings Limited, West Stone House, Berry Hill Industrial Estate, Droitwich, Worcestershire WR9 9AS, United Kingdom. This company provides magnetron sputtering services to the public using the well known PLASMAG™ magnetron. Teer Coatings Limited performed the magnetron sputtering of platinum onto carrier material in the electrode components disclosed herein in all the Examples and those depicted in the electron micrographs of Figs. 5 to 15.

In one embodiment, the magnetron configuration is a quadrupole, planar or cylindrical configuration. The sputtering may be DC or RF, at a power of from about 220W to about 400W. Bias may be from about -60V to about -140V. Gas pressure (typically Argon) may be from about 2 mTorr to about 50 mTorr. Generally, the deposition temperature is less than about 300 degrees Celsius.

Other methods of deposition may be useful in the context of the present invention. For example evaporative methods such as electron beam evaporation, thermal evaporation, flash evaporation, and resistive evaporation may be applicable. In these methods, the source material is evaporated in a vacuum. The vacuum allows vapour particles to travel directly to the target object (substrate), where the particles condense back to a solid state.

Rash evaporation techniques may be particularly applicable where a thicker film of electrode material is to be deposited.

In one embodiment, the deposition method does not rely on the autocatalysis of a deposited metal. In autocatalytic metallization (also known as electroless plating), the metal is deposited from a bath containing electrolyte, reducing agents and the reactants for autocatalytic reaction. Autocatalytic coatings have problems relating to impurity of the deposited metal, irregularity of structure, lower density due to pores and inclusions of foreign matter (such as electrolyte and chemicals used for electroless deposition), and lower electrical conductivity than bulk materials. Furthermore, autocatalytic coatings require modification of the substrate or the provision of a seed layer to facilitate the attachment of reaction products to the substrate.

A further advantage of the avoidance of autocatalytic methods is that exposure of the substrate to reactants in metallization baths (and also activation baths) is obviated. The solutions used in these baths include substances such as hydroxylamine hydrochloride, ammonia, and hydrazine monohydrate.

Hydroxylamine hydrochloride is very hazardous in case of skin contact (irritant), of eye contact (irritant), of ingestion, of inhalation. Sightly hazardous in case of skin contact (corrosive, sensitizer), of eye contact (corrosive). The amount of tissue damage depends on length of contact. Eye contact can result in corneal damage or blindness. Skin contact can produce inflammation and blistering. Inhalation of dust will produce irritation to gastrointestinal or respiratory tract, characterized by burning, sneezing and coughing. Severe overexposure can produce lung damage, choking, unconsciousness or death. This compound is known to be mutagenic for mammalian somatic cells

With respect to ammonia, mild concentrations cause conjunctivitis. Contact with higher concentrations of product will cause swelling of the eyes and lesions with a possible loss of vision. Mild concentrations of product will cause dermatitis, with higher concentrations causing caustic-like dermal burns and inflammation. Toxic level exposure may cause skin lesions resulting in early necrosis and scarring. Ammonia is corrosive and irritating to the upper respiratory system and all mucous type tissue. If it enters the deep lung, pulmonary edema will result. Pulmonary edema and chemical pneumonitis are potentially fatal conditions.

Hydrazine monohydrate causes severe eye irritation, leading in some case to irreversible eye injury. Skin irritation, skin sensitization, are seen in minor exposure, with contact of the liquid causing severe burns and ulceration. Gastrointestinal effects are seen on ingestion with symptoms of nausea, vomiting and diarrhoea being noted. In more severe episodes, digestive tract burns with immediate pain, swelling of the throat, convulsions, and possible coma ensue. The compound may cause liver and kidney damage, anemia and other blood abnormalities. Prolonged or repeated exposure may cause adverse reproductive effects and fetal effects. Hydrazine monohydrate may cause cancer according to animal studies.

Although not essential, in some embodiments of the method the recess is coated with a primer layer before the deposition step. The use of a primer layer can overcome or ameliorate any potential adhesion problems arising from the surface chemistry of the substrate. Thus, lesser consideration need be given to the surface chemistry of the silicone used as substrate. The primer layer may be a metal, and particularly a metal that is of low density, strong and corrosion resistant. Transition metals such as Titanium are particularly suitable.

It is generally desirable to avoid coating areas outside the recess formed in the carrier material with the electrode material, and so in a preferred embodiment the method comprises the step of masking the carrier material prior to deposition of the conductive material. Any suitable masking method known to the skilled person may be used in this context, however a preferred masking method comprises the application of a shadow mask to the carrier material prior to deposition of the electrode material. The function of the shadow mask is to provide a stencil through which the metal electrode material is deposited. The following publications (the contents of which are herein incorporated by reference) further describe the use of shadow masking : Ruediger Grundwald, Thin film Mirco-Optics, Elsevier 2007, Amsterdam; Dobal L Smith, Thin film deposition - Principles and Practice, McGraw-Hill, 1995.

In a preferred embodiment of the method, the mask has a thickness of between about 25 and 75 microns, more preferably between about 30 and 70 microns, more preferably between about 35 and 65 microns, more preferably between about 40 and 60 microns, more preferably between about 45 and 55 microns, most preferably about 50 microns.

The mask may be composed of any suitable material, but is preferably laser machinable. In certain embodiments of the method the mask is fabricated from a metal, and more preferably from brass. As will be appreciated, the mask is preferably fabricated from a template which is substantially flat to avoid any spaces between the mask and the carrier material that would allow entry of the conductive material during deposition.

It is desirable for the apertures in the mask to closely align with the recesses on the carrier material, and so the apertures are typically cut using a precise method such as laser machining. Indeed the use of laser ablation of the carrier material and laser machining for the mask allows for use of the same computer readable pattern for both processes. This coincidence leads to a high level of alignment between the recess and mask aperture. Indeed, placement of the mask onto the carrier material may be carried out under a stereo microscope to further improve masking accuracy. Where the carrier material is a silicone, the tackiness of the surface assists in retaining the mask. Upon placement of the mask, any air spaces between the mask and carrier material are removed by firm pressure.

After deposition of the electrode material on the carrier material and cooling to room temperature, the mask is removed. Cooling of the brass mask may lead to bending in which case it cannot be reused and must be discarded.

In some embodiments the shadow mask may be the Kapton™ tape used in the laser machining process, which may be left in place on the silicone after machining is complete. After the sputter deposition of the thick film electrode material the tape is removed. In some embodiments, removal may be facilitated with methyl siloxane. In those circumstances, the tape is removed slowly when completely immersed, thereby assisting in minimization of damage to the platinum pads or tracks deposited on the silicone carrier.

After deposition of the electrode material and removal of any mask, the electrode component comprising a carrier material bonded to an electrode material is complete. The electrode component can be incorporated into a housing or other contrivance known to the skilled artisan, or associated with other electrical hardware such as wires, circuits, and the like. In certain embodiments of the invention, the electrode component is combined with other components well known to the skilled person to provide a clinically useful device such as a cochlear implant, or a stimulating nerve cuff. The present invention provides particular advantages to cuff electrodes, considering the relative mechanical resilience of electrodes produced by the present methods. The thicker metallic pad and tracks achievable make for electrodes having improved tensile strength, relative to those provided by some or all prior artisans.

Electrode components provided by the aforementioned methods may have novel properties that provide an alternative or afford advantage over electrodes of the prior art. In a further aspect the present invention provides an electrode component comprising an electrode material portion and a carrier material portion wherein the carrier material portion is of unitary construction. An electrode having a carrier material portion that is fabricated from a single block of material (and is therefore devoid of joins) is not provided by photoresist methods of fabrication. The prior art methods require the addition of further carrier material about the deposited electrode material in order to provide a flush surface. As discussed *supra* removal of the photoresist leaves the electrode material standing proud above the surface of the carrier material. The edges of electrode material is exposed to the potential for damage, hence the need to fill in the areas left by the removal of the resist on the carrier material.

The joins or interfaces present in electrode components made by prior art methods distinguish those fabricated by the present methods, with the differences being visible by macroscopic or microscopic inspection.

A practical advantage conferred on electrodes that have a carrier or unitary construction is that they are have a greater overall mechanical stability and are more resilient to deformation. Biological electrodes are often required to be flexible in order to be properly implanted. In addition, the electrode may remain in a flexed position for many years after implantation thereby placing further stress on any joins. A carrier structure that is devoid of joins clearly has less probability of failure, and is therefore a preferred embodiment of the invention.

Another novel property of an electrode produced according to the present methods relates to roughness of the electrode material. In yet a further aspect the present invention provides an electrode component comprising an electrode material portion and a carrier material portion wherein the surface of the electrode material portion has an increased level of roughness relative to the level of roughness of a similar electrode produced by a photoresist method. The surface modification occasioned on the carrier material by a laser ablation method leads to an increase in surface roughness. This is a departure from the smooth electrode surfaces typical for sputtered films.

Of practical importance, this increased roughness may translate to an increase in electrode material surface area. The increase in surface area in turn allows for electrode pads of a smaller size, and the ability to therefore implement higher electrode densities in multi-electrode arrays such as those necessary in cochlear implants. Thus, in a further aspect the present invention provides a multi-electrode array for use in a cochlear implant, the array comprising at least about 22, 23, 24, 25, 26, 27, 28, 29, 30, 31, or 32 electrodes.

Quite apart from the advantage in electrode density, smaller electrodes in cochlea implants can be advantageous in the overall size of the implant is smaller. A further aspect of the present invention therefore provides an electrode component comprising an electrode material portion and a carrier material portion wherein the electrode material portion has a geometric surface area of less than about 0.03 mm², 0.0299 mm², 0.02298 mm², 0.02297 mm², 0.02296 mm², or 0.02295 mm².

As used herein, the term "geometric surface area" means the surface area as calculated on the assumption that the surface is completely planar. For example, if the electrode were a rectangle the geometric surface area would be calculated by simply multiplying the length by width.

Smaller electrodes may facilitate greater insertion depth of the implant. The length of the human cochlea is 33 to 36 mm, with some the implants not capable of reaching the apical tip when inserted. This physical limitation can mean that it is not possible to stimulate the higher frequencies about the apical tip of the cochlea.

Smaller electrodes may also be capable of more accurate spatial stimulation of a tissue. A problem in cochlear implants is that the electrical signals tend to fan outwards from the electrodes. This can result in some regions of the cochlear receiving errant stimulation or receiving overlapping signals. Smaller electrodes can be placed closer together providing the potential benefit of decreased signal overlap. In a further aspect the present invention provides a multi-electrode array for use in a cochlear implant, the array comprising a plurality of electrode pads, the distance between two pads being less than about 0.7, 0.6, 0.5, 0.4, 0.3, 0.2, or 0.1 mm.

As mentioned supra, an advantage of the present methods over the prior art photolithographic methods is that thicker films of electrode material can be deposited. Accordingly, a further aspect of the present invention therefore provides an electrode component comprising an electrode material portion and a carrier material portion wherein the electrode material portion has a thickness of greater than about 1 , 2, 3, 4, 5, 6, 7, 8, 9, 10, 11 , 12, 13, 14, 15, 16, 17, 18, 19 or 20 micron. It is proposed that the present methods allow for the first time a combination of thick electrode films with a biocompatible carrier material. In some embodiments the thicker films of the present invention have the practical advantages of greater mechanical stability and a higher level of machinability.

The present invention has the further advantage in that electrode pads as well as tracks can be formed on the substrate. Some methods of the prior art are only suitable for forming tracks, which connect the main electrode pad to other components, such as connecting means. The ability to fabricate both tracks and pads in a single step is of clear advantage in terms of simplicity and cost of manufacture.

Also described herein is an electrode component produced according to any of the methods of the present invention.

Described herein for information purposes only is a method of treating, preventing or ameliorating a condition associated with aberrant electrical stimulation of a tissue, the method comprising the step of implanting an electrode component, multi-electrode array or implantable electrode device into an animal in need thereof. Many such conditions are known in medicine and the veterinary sciences, however one embodiment of the method provides for the treatment of deafness which has been successfully accomplished by implantation of a cochlear electrode array.

The method of the present invention will now be more fully described by reference to the following nonlimiting examples.

### EXAMPLES

### EXAMPLE 1 : Fabrication of electrode component using a silicone as the carrier and platinum as the conductive material.

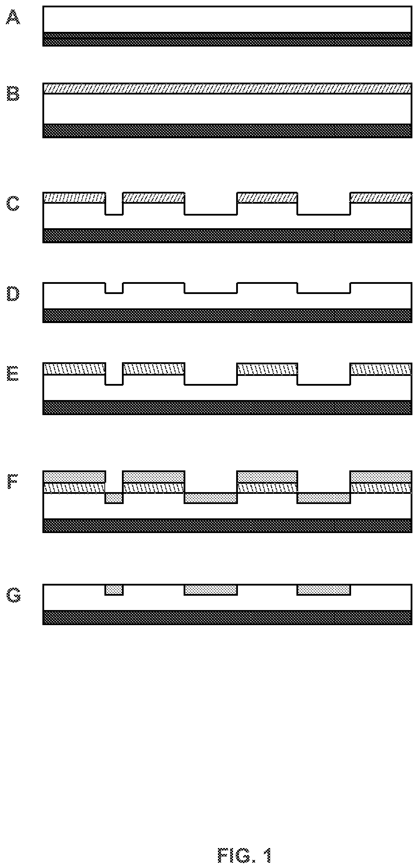

Silicone elastomer MED 4860 was mixed, diluted in heptane 40% by weight before spin coating onto a glass wafer (Fig.1a).

A piece of the substrate was placed onto the chuck of the spin coater and the surface of the substrate covered with silicone using a pipette.

The spin coating rate and duration was adjusted to the viscosity of the silicone to obtain required thickness of about 15 to 20 micron. For MED 4860 silicone diluted with 40% heptane the following conditions were used: 2000rpm for 20 seconds to obtain a 15 micron thick layer. After spin coating the sample was placed immediately on a hotplate set to 100°C and left to evaporate the solvent for 20 minutes which is sufficient to obtain a cured silicone film. Where required, the spin coating can be repeated several times to build up thicker film.

When the desired film thickness was reached the silicone film was peeled from the substrate. However, the film requires a firm substrate for further processing, and was therefore reattached to the glass wafer on which it was spin coated. This re-attachment was achieved by virtue of the tackiness of silicone which may be easily removed after processing. Immediately after re-attaching the silicone film to the substrate, Kapton™ tape was laid on the surface onto the top surface of the silicone. Any air bubbles were removed with gentle pressure.

The substrate-silicone-tape composite was then laser machined using an ArF excimer laser with the wavelength 193 nm. The laser machining was performed from the top of the composite, and through the kapton™ tape protective layer leading to a recess being formed into the silicone, the recess having a depth of 10 micron. After laser machining the Kapton™ tape was peeled off.

A shadow mask to overlay the silicone was fabricated by laser machining brass foil having a thickness of 50 micron. The mask has an identical patterning as that used in the machining of the recesses in the silicone carrier.

The laser machining of the mask was performed using the 4^{th} harmonic of a Nd:YAG laser with a wavelength of 266 nm. The duration of the laser pulses was 15 ns. The beam was brought to focus on the target substrate with a 5x objective lens, producing a spot size of 6 µm. The brass masks were produced with a repetition rate of 500 Hz, and a corresponding average power of 50 mW. A summary of the processing conditions is shown in Table 1, *infra.*

**Table 1: Laser machining parameters for the brass shadow mask**

| Material | Pulse (ns) | Pulse rep rate (Hz) | Power (mW) | Energy (uJ) | Spot size (µm) | Fluence (J/cm2) | Passes | Pass rate (mm/min) |
|---|---|---|---|---|---|---|---|---|
| Brass | 15 | 500 | 50 | 100 | 6 | 350 | 5 | 50 |

As mentioned, the brass mask and the laser machined silicone carrier share the same patterning. Accordingly, the mask is placed on top of the laser machined silicone such that the apertures of the mask are in register with the recesses of the silicone. The placement was done manually under a stereographic microscope for accuracy. Once the mask was aligned with the laser machined pattern in silicone, the mask was firmly pressed onto the silicone to remove any air bubbles or gaps between the mask and the silicone.

The masked silicone was then loaded into the chamber of the magnetron sputtering equipment. To deposit a thick film, a thin titanium adhesion layer of about 50 nm was first sputter deposited onto the masked silicone by Teer Coatings Ltd (United Kingdom). Without exposing the masked silicone to air, a 10 micron thick layer of platinum was deposited. The sputtered and masked silicone was removed from the chamber and left to cool to room temperature. During cooling the mask bent due to thermal stress, and detached from the masked silicone to expose the platinum pattern.

A diagrammatic representation of the method is shown in Fig. 1: a) glass coated with silicone, had (b) Kapton™ tape adhered to the silicone. (c) The Kapton™ tape and silicone was laser machined, and (d) the tape subsequently removed and replaced by (e) a brass mask. (f) The surface of the exposed silicone and brass was then coated with platinum (Pt) by a sputtering method. The uppermost layers of brass and Pt were removed.

### EXAMPLE 2: First alternative method ("design A") of fabrication of electrode component using a silicone asthe carrier and platinum asthe conductive material

The electrode component in this Example was prepared in a similar manner to that disclosed in Example 1, except that the silicone carrier was solvent casted from a silicone solution. A mould was fabricated from Teflon, into which a silicone solution was poured. The solution was prepared at a lower viscosity compared to that normally used for spin coating.

After casting, any solvent was left to evaporate and the silicone left to cure. Curing was accelerated by heating to 90°C under which conditions curing was achieved by 30 minutes. After curing and cooling down the sheet is peeled off from the mould.

A further difference in the electrode component produced in this example is that a protective layer of silicone was used in place of the Kapton™ tape, and a silicone mask was used instead of the brass foil disclosed in Example 1.

The protective layer of thin silicone was formed by spin coating, and had a thickness of about 70 micron, although thinner layers will be operable. It will also be noted that a casting method may be used in place of spin coating. When placing the protective sheet on top of silicone substrate a film of isopropanol was found to be useful as it enables sliding of the two sheets relative to each other. Alternatively, 100%ethanol may be used.

The silicone shadow mask was fabricated by laser micromachining using the 4^{th} harmonicof a Nd:YAGlaser with a wavelength of 266 nm. The duration of the laser pulses was 15 ns. The beam was brought to focus on the target substrate with a 5x objective lens, producing a spot size of 6 µm. The silicone masks were fabricated using a pulse repetition rate of 2000 Hz, with an average laser power of 50 mW.

**Table 2: Laser machining parameters for all silicone mask fabrication**

| Material | Pulse (ns) | Pulse rep rate (Hz) | Power (mW) | Energy (uJ) | Spot size (µm) | Fluence (J/cm2) | Passes | Pass rate (mm/min) |
|---|---|---|---|---|---|---|---|---|
| Slicone | 15 | 2000 | 50 | 25 | 6 | 90 | 5 | 50 |

To prevent the mask from overly flexing a thin gold film of around 50 nm thickness was sputter coated on the silicone mask. This film provides for a sturdier mask. The mask was aligned on the target using isopropanol to enable gliding of two silicone sheets relative to each other with the silicone side in contact with the silicone substrate.

### EXAMPLE 3: Second alternative method ("design B") of fabrication of electrode component using a silicone asthe carrier and platinum as the conductive material.

In addition to methods described in Examples 1 and 2 the silicone substrate can be modified to produce higher surface areas by laser machining of the silicone. In this example, fused silica content in the silicone is increased. Alternatively, any other organic or inorganic (non-metallic) nano-or micro particles may be used as filler. The formulation and fraction of the filler is adjusted so that the silicone retains the required flexibility. The particles are evenly distributed within the matrix, and when laser machining the silicone these particles are not ablated by the laser. As a result, domes are formed in the silicone carrier. Because the particles are not firmly embedded in the silicone matrix they are washed out so that only silicone remains on the surface. These domes increase the surface area of the substrate which is retained or further increased after platinum sputtering.

### EXAMPLE4: Insulation of metal electrode material.

Insulation of the tracks and insulation of the edges of the platinum pads may be required to prevent high density discharge at the edges. To achieve this, a further thin layer of silicone was spin coated on the surface of the silicone-metal composite prepared according to Example 1. This further layer was applied to achieve a thickness of about 25 micron (thicknesses of 10 to 40 micron being generally accepted).

The electrode pads were exposed by a further excimer laser machining step. Prior to laser machining, a protective layer of Kapton™ tape was placed on top of the further layer of silicone. Laser machining was then performed only on top of the electrode pads until the entire thickness of the further silicone layer was removed leaving the platinum electrode pads exposed.

### EXAM PLE5: Testing of electrode device having thick platinum film

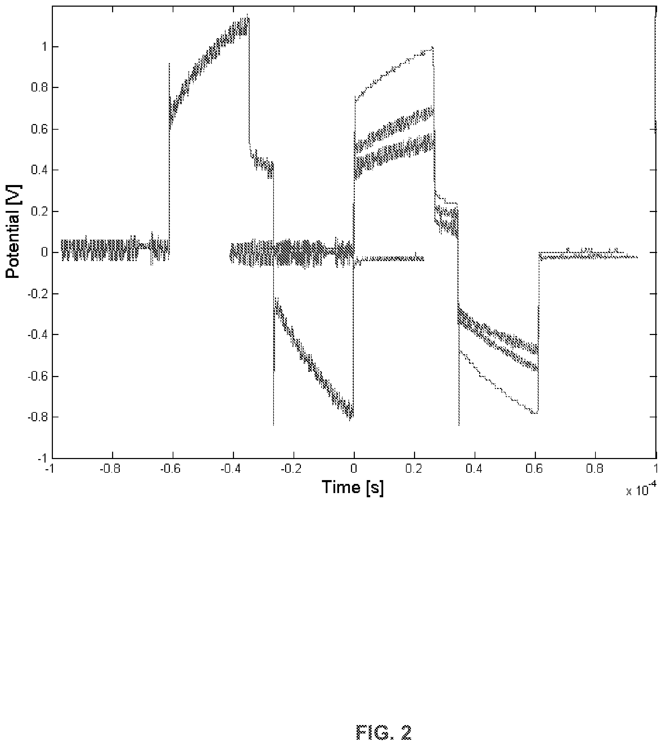

A typical test for cochlear implants is the potential transient measurement, which represents the voltage response of the electrode *in vitro* after a specific current pulse was applied to the electrode. Fig. 2 shows the biphasic voltage pulse applied to three different types of electrodes, guinea pig test electrode (this corresponds to the classic cochlear implant electrode), a thick platinum film which was deposited on a substrate that was not laser machined and on two samples that were laser machined according to the present methods before the platinum thick film was deposited.

The data shows that laser machined electrodes typically performed better than the guinea pig electrode (green graph compared to pink graph). Comparing the red graph to the black graph shows that the surface roughness (as seen in the laser machined samples) provides superior performance over smooth thick film samples that have low surface roughness.

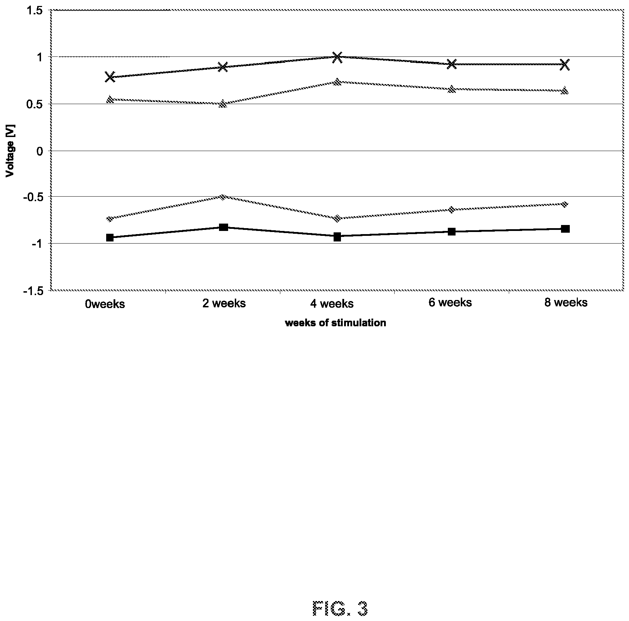

The data in Fig. 3 was collected on one laser machined electrode which was stimulated with a current of 1.75 mA, pulse duration 25 microseconds and the rate of 600 pulses per second. A charge balanced biphasic pulse and monopolar stimulation was applied. The electrolyte was phosphate buffered saline. It can be seen from the data that the voltage remains substantially steady over the stimulation period indicating high chemical stability of the thick film.

### EXAM PLE6: Functional comparison of thick film electrode to thin film electrode.

The thick film electrode fabricated accordingly to the method shown in Fig. 1 was compared to a thin film electrode made with photolithography patterning on glass substrate. The thin film electrode had a geometric area of 0.0045 cm² compared to 0.03 cm² for a thick film electrode. Thus the geometric area of the thin film electrode is 50% higher than the geometric area of the thick film. However, the surface roughness is very low for the thin film electrode with Rₐ around 4 nm. In contrast, the thick film electrode has high surface roughness which leads to higher real surface area and in turn leading to the better performance of the thick film electrode. Better performance can be measured on lower voltage at the end of the pulse for thick film electrode. This also implies that the polarisation impedance is lower for the thick film electrode as the waveform for the thick film appears flatter.

### EXAMPLE 7: Structural characterization of platinum thick film electrode by scanning electron microscopy.

The carrier/electrode material composites shown in this Example were produced by the method as shown in Fig.1 herein, except where the electrode material was deposited onto glass. All platinum thick films in this example were deposited by magnetron sputtering performed by Teer Coatings Limited (United Kingdom) onto a silicone carrier (or glass, where indicated).

### (i) Topographical characterisation.

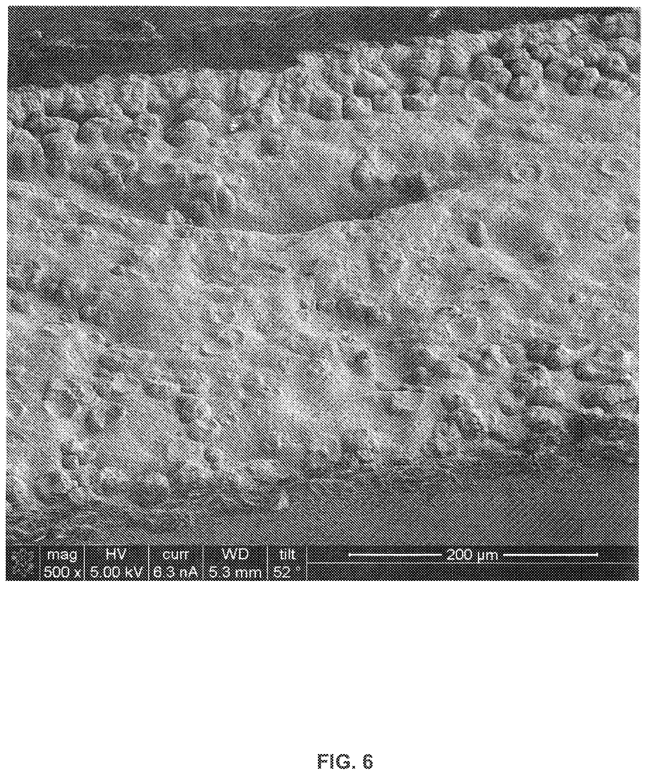

Fig. 5 shows a thick film sample where the silicone substrate was laser machined. Five contact pads are shown, with several associated tracks. A magnification of the one of the contact pads is presented in Fig. 6 showing the surface of the thick film.

It will be noted that the surface of the platinum thick film demonstrates roughness. The surface roughness may be divided into two categories. One category concerns the roughening of the silicone substrate due to laser machining which results in a rough platinum film.

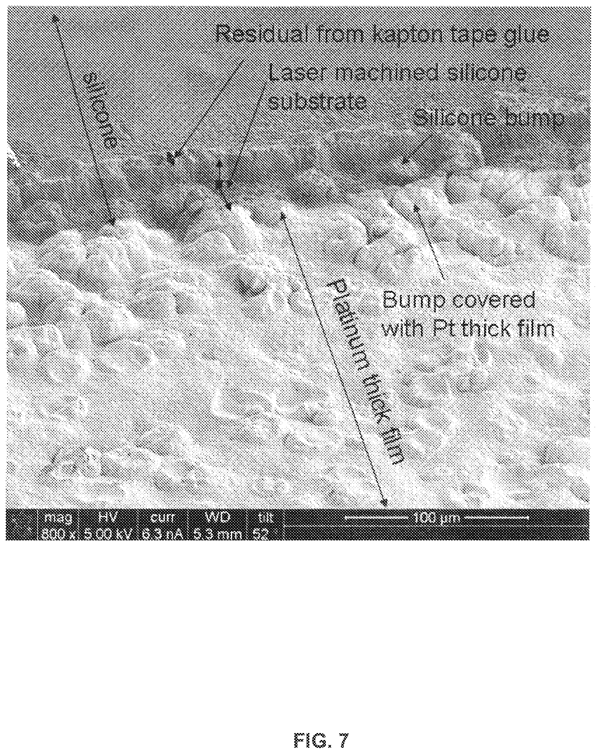

The other category is related to the presence of bumps (shown more clearly in Figs. 7, 8 and 9) which are the result of the presence of residual Kapton™ glue tape. Although the bumps are also related to the laser machining process, their origin is due to the faults in machining the Kapton™ tape and not so much the silicone substrate itself. These bumps are especially large around the edges of the samples where their density was greatest for this sample.

In the central parts of the pads, bumps having a smaller diameter and lower height were observed. Also the areas of the film without bumps can be dearly identified as wavy and rough in Fig. 6.

Further evidence for the bumps being remains of the Kapton™ tape glue is shown in Fig. 7. This figure shows the edge of one of the pads where the film was not sputtered into the whole laser machined recess. The darker areas represent silicone whereas the bright areas of the image show platinum thick film. The rough laser machined silicone substrate is visible in Fig. 7 compared to smooth silicone further away from the laser machined site. There is some Kapton™ tape glue around the edges of the laser machined site. Several bumps in the laser machined silicone area are visible. Bumps of the same shape and similar size can be observed all over the surface covered with platinum film.

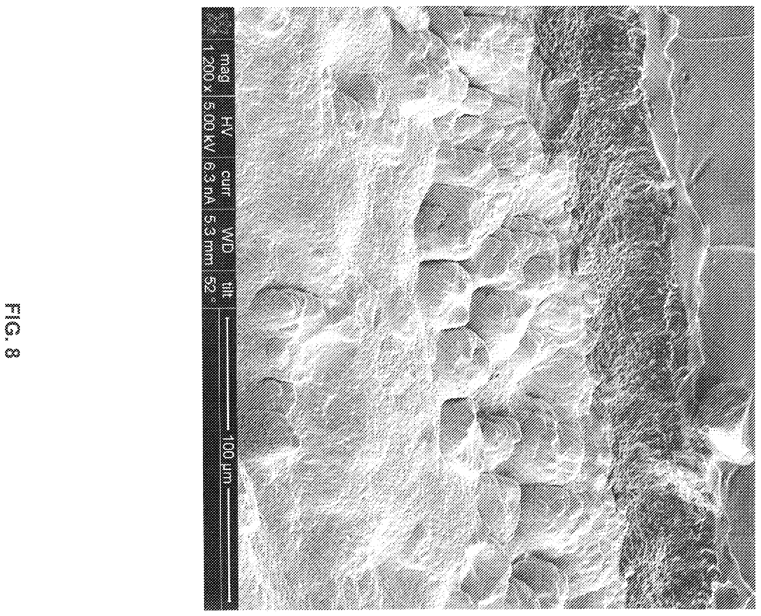

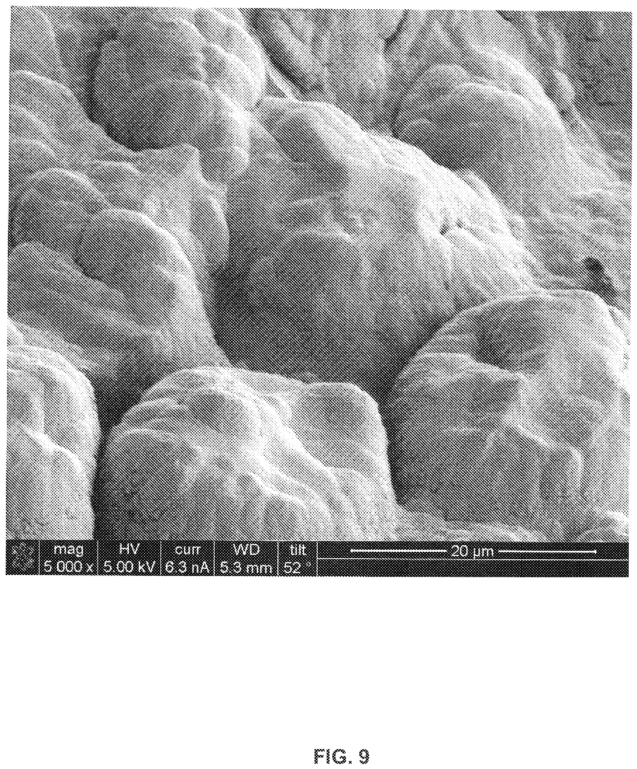

Another image of a different sample area showing the edge of the pad with some of silicone exposed and stronger bumps around edge is presented in Fig. 8. A magnification of the bumps covered with platinum film is shown in Fig. 9 showing the fractal-like surface of the bumps.

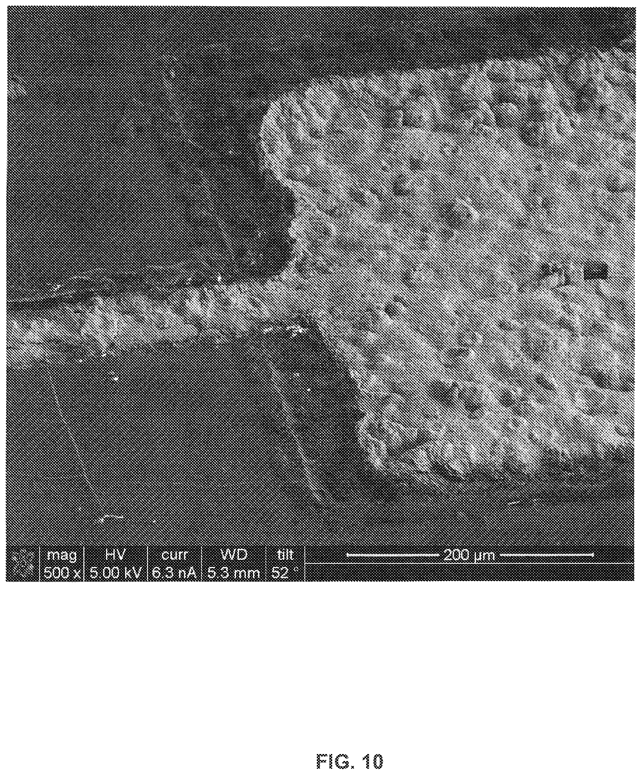

Exposure of laser machined silicone in this sample was due to a misalignment of the shadow mask. The mask was misaligned by around 50 microns and parts of the laser machined silicone pad were not sputtered with the thick film, Fig. 10.

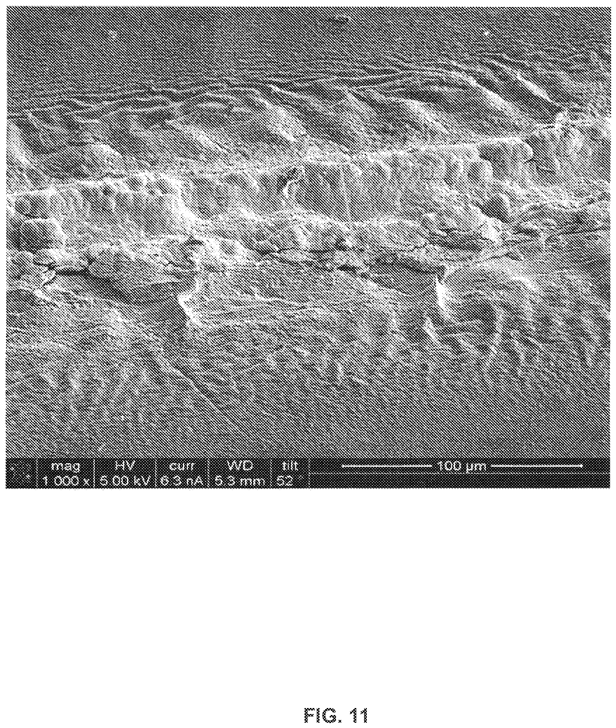

Fig.11 shows a track with the platinum sputtered width around 59 micron (arrow) and the bulged silicone and glue present around the track.

### (ii) Cross-sectional characterization.

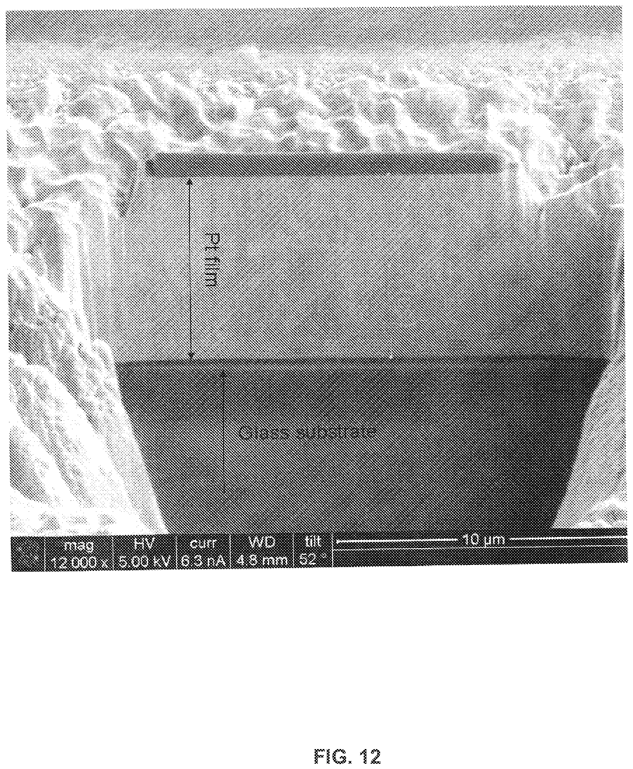

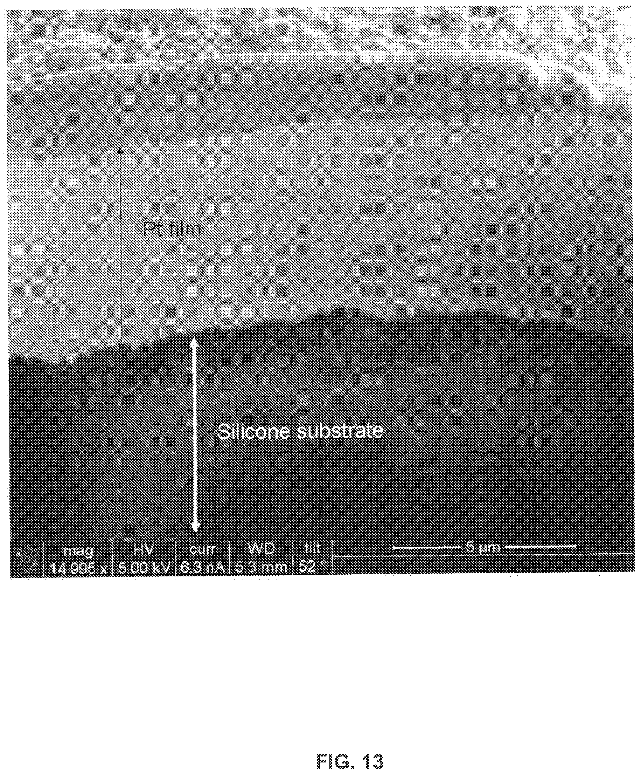

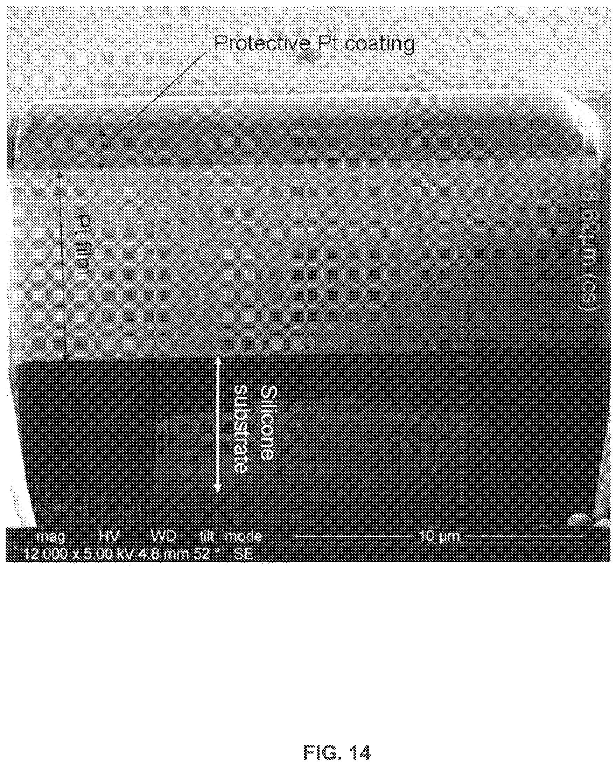

Cross sections of various carrier/electrode material composites were cut using Focused Ion Beam (FEI Nova Dual beam). Three samples were chosen:
- platinum film on glass substrate (Fig. 12)
- platinum film on laser machined substrate (Fig. 13), and
- platinum film on not laser machined substrate (Fig. 14).

All samples had a protective Pt coating deposited using ion beam. The thickness of this layer was around 2 µm and the contrast between the Pt thick film and the protective Pt coating through the impurities in the protective coating.

All cross section images show dense thick film without any pores. The substrate-film interface differs between the samples. For platinum deposited on glass (Fig.12) and on silicone (Fig. 14) that has not been laser machined, the interface between platinum and the substrate is as expected, very smooth and even. In contrast, if the film was deposited on laser machined silicone (Fig. 13) the interface is rough and has some curvature. Also, some pores can be observed in the silicone, most likely due to laser machining. The curvature of the substrate is on microscopic scale thus it is likely that the curvature is also due to laser machining.

Measurements of the film thickness have led to different results. Between the samples from the same batch (films deposited on silicone) thickness measurements ranged from 6.9 µm to 8.6 µm. Some thickness variation was observed in the cross section of the film deposited on laser machined substrate. As can be seen in Fig. 13 the film thickness is not uniform and can differ locally. The contour of the Pt/silicone interface is not mirrored in the platinum surface. In the centre of the cross section the thickness is slightly lower than at the edges of the milled cross section. Sputtered films usually adapt to the substrate and give uniform thickness throughout the film. However, this property was only found on laser machined silicone and not if Pt was deposited on not laser machined silicone. Thus, the non-uniform film thickness on laser machined substrate can be also linked to the laser machining of the substrate.

Other reasons for thickness variation between the samples deposited in one batch might be the non-uniformity of the sputtering process itself. It is likely that, for instance, on a 4" wafer the thickness of the coated layer can differ up to 10% between the centre of the wafer and the edges of the wafer. Although all samples were guaranteed to have uniform coating the thickness for samples having different positions in the chamber might vary slightly, so that a1 µm difference between the samples is possible.

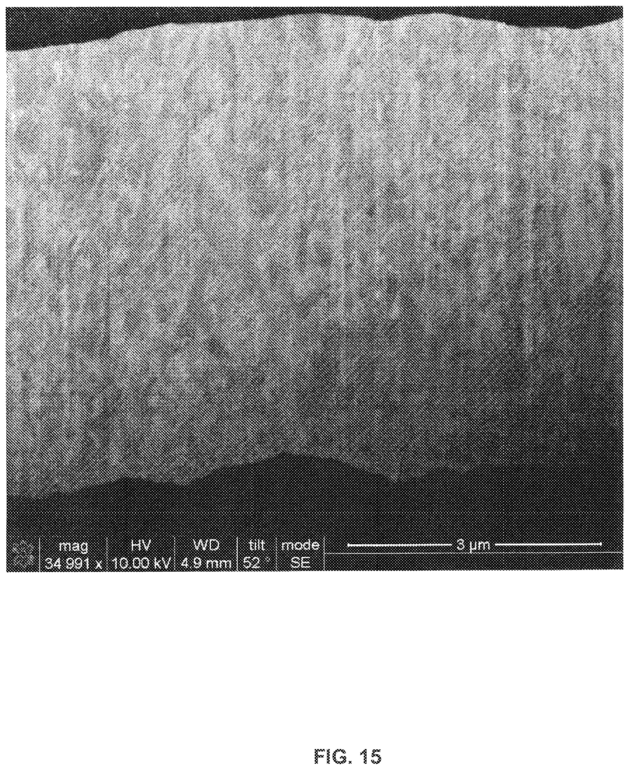

A magnification image of the cross section showing the crystallites (or grains) to some extent is presented in Fig. 15. The contrast in the images stems from the different orientation of the grains. The sputtered films usually demonstrate many defects and it is possible that the orientation and crystallite size can vary depending on the defects in the lattice. Although the X-ray Diffraction (XRD) has shown that the film is highly (111) orientated and thus has columnar texture, the columns can be rotated around their axes and lead to a different contrast in the cross section. Further, as can be seen in Fig. 15 it is unlikely that columns are more than 1 micron tall. To illustrate, in thin films usually the columnar microstructure is associated with columns that run from the bottom interface to the top interface. However, for very thick films this is not the case.

From the cross section images it is proposed that the pores may not be responsible for the depth profile of the mechanical properties in the films. The grain size distribution or thin oxide layer is more likely to be responsible for the depth profile of the mechanical properties.

## Claims

1. A method for fabricating an electrode device component, the method comprising the steps of:
(i) providing a biocompatible carrier material having a protective layer and
(ii) performing an ablative method on the biocompatible carrier material to form a recess, the recess capable of receiving a biocompatible electrode material having a thickness of at least about 1 micron,
(iii) removing the protective layer, and
(iv) depositing a conductive material having a thickness of at least about 1 micron into the recess of the carrier material,
wherein the ablative method does not require exposure of the carrier material to a chemical compound; and
**characterised in that** the protective layer is provided by applying a pre-formed film to the biocompatible carrier material.

2. A method according to claim 1 wherein the ablative method relies on heating the carrier material; preferably wherein the ablative method is a laser ablative method.; preferably wherein the laser ablative method comprises use of an excimer laser.

3. A method according to any one of claims 1 to 2 wherein the recess formed by the ablative method is of sufficient depth to allow the deposition of an electrode film of between about 1 and 25 micron thickness.

4. A method according to any one of claims 1 to 3 comprising the step of applying the protective layer to the carrier material after the step of providing the carrier material and before the step of performing the ablative method.

5. A method according to claim 4 wherein the protective layer has a thickness of less than about 10 micron; preferably wherein the film has a thickness of from about 5 to 7 micron; preferably wherein the film is a polymeric film; preferably wherein the polymeric film is a polyimide; preferably wherein the conductive material is deposited using a vapour deposition method; wherein the vapour deposition method is a sputtering method, the method preferably comprising the step of masking the carrier material prior to deposition of the conductive material.

6. A method according to claim 5 comprising the step of applying a shadow mask to the carrier material prior to deposition of the electrode material; preferably wherein the shadow mask has a thickness of between about 25 and 75 microns; preferably wherein the shadow mask is fabricated from a laser machinable material.

## Patentansprüche

1. Herstellungsverfahren für eine Elektrodenkomponente, wobei das Verfahren die folgenden Schritte umfasst:
(i) Bereitstellen eines biokompatiblen Trägermaterials mit einer Schutzschicht und
(ii) Durchführen eines ablativen Verfahrens am biokompatiblen Trägermaterial, um eine Ausnehmung auszubilden, wobei die Ausnehmung ein biokompatibles Elektrodenmaterial mit einer Dicke von mindestens etwa 1 Mikron aufnehmen kann,
(iii) Entfernen der Schutzschicht, und
(iv) Ablagern eines leitfähigen Materials mit einer Dicke von mindestens etwa 1 Mikron in der Ausnehmung des Trägermaterials,
worin das ablative Verfahren nicht das Aussetzen des Trägermaterials an eine chemische Verbindung erfordert; und
**dadurch gekennzeichnet, dass** die Schutzschicht durch Auftragen eines vorgeformten Films auf das biokompatible Trägermaterial bereitgestellt wird.

2. Verfahren nach Anspruch 1, worin sich das ablative Verfahren auf das Erhitzen des Trägermaterials stützt; vorzugsweise, worin das ablative Verfahren ein laserablatives Verfahren ist; vorzugsweise, worin das laserablative Verfahren die Verwendung eines Excimerlasers umfasst.

3. Verfahren nach einem der Ansprüche 1 bis 2, worin die vom ablativen Verfahren ausgebildete Ausnehmung tief genug ist, um die Ablagerung eines Elektrodenfilms mit einer Dicke zwischen etwa 1 und 25 Mikron zu erlauben.

4. Verfahren nach einem der Ansprüche 1 bis 3, umfassend den Schritt des Auftragens der Schutzschicht auf das Trägermaterial nach dem Schritt des Bereitstellens des Trägermaterials und vor dem Schritt des Durchführens des ablativen Verfahrens.

5. Verfahren nach Anspruch 4, worin die Schutzschicht eine Dicke von weniger als etwa 10 Mikron aufweist; vorzugsweise, worin der Film eine Dicke von etwa 5 bis 7 Mikron aufweist; vorzugsweise, worin der Film ein polymerer Film ist; vorzugsweise, worin der polymere Film ein Polyimid ist; vorzugsweise, worin das leitfähige Material unter Verwendung eines Dampfablagerungsverfahrens abgelagert wird; worin das Dampfablagerungsverfahren ein Sputterverfahren ist, wobei das Verfahren vorzugsweise den Schritt des Maskierens des Trägermaterials vor Ablagerung des leitfähigen Materials umfasst.

6. Verfahren nach Anspruch 5, umfassend den Schritt des Auftragens einer Schattenmaske auf das Trägermaterial vor Ablagerung des Elektrodenmaterials; vorzugsweise, worin die Schattenmaske eine Dicke zwischen etwa 25 und 75 Mikron aufweist; vorzugsweise, worin die Schattenmaske aus einem laserbearbeitbaren Material hergestellt ist.

## Revendications

1. Un procédé destiné à la fabrication d'un composant de dispositif d'électrode, le procédé comprenant les étapes consistant à :
(i) prévoir un matériau de support biocompatible présentant une couche de protection et
(ii) exécuter un procédé ablatif sur le matériau de support biocompatible pour former un évidement, l'évidement étant capable de recevoir un matériau d'électrode biocompatible ayant une épaisseur d'au moins environ 1 micron ;
(iii) retirer la couche de protection, et
(iv) déposer un matériau conducteur ayant une épaisseur d'au moins environ 1 micron dans l'évidement du matériau de support,
le procédé ablatif ne nécessitant pas l'exposition du matériau de support à un composé chimique ; et
**caractérisé par le fait que** la couche de protection est fournie par l'application d'un film préformé sur le matériau de support biocompatible.

2. Un procédé selon la revendication 1 dans lequel le procédé ablatif repose sur le chauffage du matériau de support ; de préférence dans lequel le procédé ablatif est un procédé ablatif par laser ; de préférence dans lequel le procédé ablatif par laser comprend l'utilisation d'un laser excimère.

3. Un procédé selon une quelconque des revendications 1 à 2 dans lequel l'évidement formé par le procédé ablatif a une profondeur suffisante pour permettre le dépôt d'un film d'électrode d'une épaisseur entre environ 1 et 25 microns.

4. Un procédé selon une quelconque des revendications 1 à 3 comprenant l'étape consistant à appliquer la couche de protection sur le matériau de support après l'étape consistant à prévoir le matériau de support et avant l'étape consistant à exécuter le procédé ablatif.

5. Un procédé selon la revendication 4 dans lequel la couche de protection a une épaisseur inférieure à environ 10 microns ; de préférence dans lequel le film a une épaisseur d'environ 5 à 7 microns ; de préférence dans lequel le film est un film polymère ; de préférence dans lequel le film polymère est un polyimide ; de préférence dans lequel le matériau conducteur est déposé à l'aide d'un procédé de dépôt en phase vapeur ; dans lequel le procédé de dépôt en phase vapeur est un procédé de pulvérisation cathodique, le procédé comprenant de préférence l'étape consistant à masquer le matériau de support avant le dépôt du matériau conducteur.

6. Un procédé selon la revendication 5 comprenant l'étape consistant à appliquer un masque d'ombre sur le matériau de support avant le dépôt du matériau d'électrode ; de préférence dans lequel le masque d'ombre a une épaisseur d'entre environ 25 et 75 microns ; de préférence dans lequel le masque d'ombre est fabriqué en matériau usinable au laser.
